# EUROPEAN PATENT APPLICATION

(11) **EP 0 622 064 A2**
(43) Date of publication of application: **02.11.1994**
(21) Application number: 94106679.7
(22) Date of filing: 28.04.1994
(51) Int. Cl.: A61F 13/15

(54) **Hygienic absorbent article**

(30) Priority: 28.04.1993 US 55053; 28.04.1993 US 55052
(71) Applicant: KIMBERLY-CLARK CORPORATION, Neenah, Wisconsin 54956-0349 (US)
(72) Inventor: Garvey, Michael Joseph, Appleton, Wi 54911 (US); Fell, Joseph Patrick, Neenah, WI, 54956 (US); McFarland, Timothy Maurice, Neenah, WI, 54956 (US); Anderson, Gary Chester, Appleton, WI 54915 (US); Mitchler, Patricia Ann, Neenah, WI 54956 (US)
(74) Representative: DIEHL GLAESER HILTL & PARTNER

(57) **Abstract**

An absorbent article (10) is disclosed having a liquid-permeable cover (12), an absorbent (17), a liquid-impermeable foam baffle (20), and a layer of hot melt adhesive (19) bonding the absorbent (17) to the foam baffle (20). The baffle (20) is a polyolefin foam having a thickness ranging from about 0.51 millimeters to about 1.54 millimeters and having a density ranging from about 0.0225 g/cm³ to about 0.0962 g/cm³.

## Description

This invention relates to an absorbent article.

Disposable absorbent articles, such as sanitary napkins catamenial pads, panty liners, training pants, incontinent garments, diapers, and the like are designed to be worn adjacent to a human body to absorb discharged body liquids. Conventionally, disposable absorbent articles comprise an absorbent pad positioned between a liquid-permeable nonwoven top sheet and a liquid-impermeable back sheet. The nonwoven top sheet draws the waste away from the contacting skin and into the absorbent core while the liquid impermeable back sheet prevents the absorbed liquids from leaking out of the absorbent article. Numerous variations of, and elements in addition to, these basic components have been taught with each variation or additional element being directed to improving specific characteristics of the article. Regardless of the particular construction, however, it is expected that the absorbent article will have characteristics which permit liquid to penetrate the liquid-permeable top sheet while quantities of liquid are absorbed by the core.

The body fluid which penetrates the thickness of the core will be prevented from wetting the surrounding undergarment by a liquid- impermeable baffle. Usually, the baffle is a polyethylene film having a thickness of about .025 to .13 millimeters (about .001 to about .005 of an inch). Optionally, the absorbent article may provide for a pressure sensitive adhesive applied to the garment facing side of the baffle. The adhesive is of the type that is capable of securing the absorbent article to the crotch portion of an undergarment, yet permitting removal of the article when soiled.

For convenience in the description, a panty liner or a very thin absorbent pad used primarily for feminine hygiene, which may be used for undergarment protection between menstrual periods as well as absorbing light flow between and during periods will be described herein. However, it is to be understood that this preferred embodiment of this invention is not limiting the invention only to panty liners.

Heretofore, in constructing a panty liner, some means was provided for holding the fibers constructing the absorbent core together to form a layer so the laminate does not fall apart and/or for bonding the layer of fibers to adjoining layers.

Several approaches have been taken in the prior art. One technique is to seal the fibers within an envelope. The envelope is formed by sealing or adhesively attaching the cover sheet and the baffle together about their perimeters. These layers are typically a nonwoven material and are bonded to each other by application of heat or with adhesive.

Another approach has been to add thermoplastic powders, fibers, or fibrils to the fiber layer and to apply heat during lamination to form a supporting web of thermoplastic material throughout the fibrous layer as well as a bond between the thermoplastic web and adjoining thermoplastic liquid-permeable and liquid-impermeable layers. Typically, the laminate is embossed during application of heat so that bonding occurs primarily in embossed areas.

Crosslinkable latex adhesives have also been employed in bonding the absorbent fibers together and to bond the adjacent layers together. One problem in using latex adhesives in this manner is that the adhesive is at least partially absorbed into the absorbent fibers. This can reduce the efficiency of the absorbent fibers. Another problem with using a latex adhesive to bond the adhesive fibers together is that upon drying the absorbent fibers have an increased stiffness, resulting in an article that is less comfortable to the wearer.

Embossing has also been used in such constructions to cause the latex adhesive to migrate into the embossed layers prior to curing.

Other patent literature discloses a pad having the absorbent fibers enclosed in a tissue or a nonwoven wrap. The edges are sealed by highly compressing the pad around its entire periphery. However, when the panty liner's entire periphery is compressed or compacted sufficiently to seal the edges, relatively harsh and sharp edges are formed which tend to be abrasive.

It is therefore desirable in the art to produce a panty liner that would have a low cost for manufacturing and would be soft and comfortable to the wearer yet resilient and absorbent in use.

This object is solved by the absorbent article of any one of independent claims 1, 3 and 14. Further advantageous features, aspects and details of the absorbent article are evident from the dependent claims, the description and the drawings. The claims are intended to be understood as a first non-limiting approach of defining the invention in general terms.

The invention provides an absorbent article having a foam baffle.

The invention provides especially a disposable absorbent article having a liquid-permeable cover sheet, a foam baffle, and an absorbent therebetween. More particularly, this invention provides according to a specific aspect an absorbent article that uses a foam as a liquid-impervious baffle and hot melt adhesive to bond the foam to the absorbent.

According to a preferred aspect, this invention relates to an absorbent article having a liquid-permeable cover sheet; an absorbent; a liquid-impermeable foam baffle having a thickness ranging from about 0.51 millimeters to about 1.54 millimeters, a density of from about 0.0225 g/cm³ to about 0.0962 g/cm³, and having an absorbent facing surface and a garment facing surface; and a layer of hot melt adhesive applied to the absorbent facing surface to bond the absorbent to the foam.

In a preferred embodiment, the absorbent article is a thin elongated panty liner in which a polyethylene foam baffle is utilized as a liquid-impervious backing. The cover sheet, the absorbent core, and the foam baffle can have coterminous edges.

The completed panty liner may be used in combination with a tight fitting undergarment without supplementary attachment means, if desired. Preferably, a pressure sensitive, garment attachment adhesive is disposed on a least a portion of the garment facing surface of the polyolefin foam baffle and covered with a removable peel strip which may be peeled off by the user for more positive attachment purposes.

A general aspect of this invention is to provide an absorbent article utilizing a polyolefin foam as a liquid-impermeable baffle. A more specific aspect of this invention is to provide a panty liner that is simpler, less expensive to manufacture, and has greater comfort to the wearer. Another aspect of the invention provides an absorbent article which is softer and provides improved fluid management.

Still other aspects and advantages of the present invention will become more apparent to those skilled in the art in view of the following description and the accompanying drawings.

FIG. 1 is a plan view of an absorbent article such as a panty liner.

FIG. 2 is an enlarged sectional view taken along 2--2 of FIG. 1.

FIG. 3 is a perspective view of the bottom of the absorbent article showing a garment adhesive strip.

FIG. 4 is schematic of a method for producing the absorbent article shown in FIG 1.

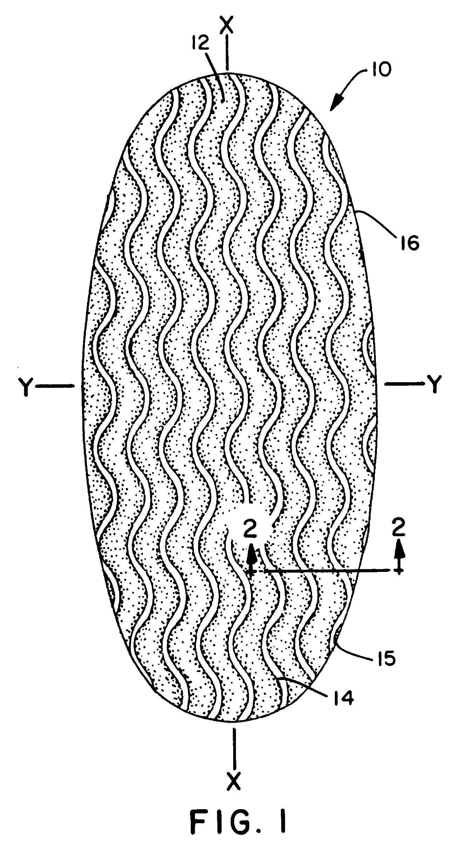

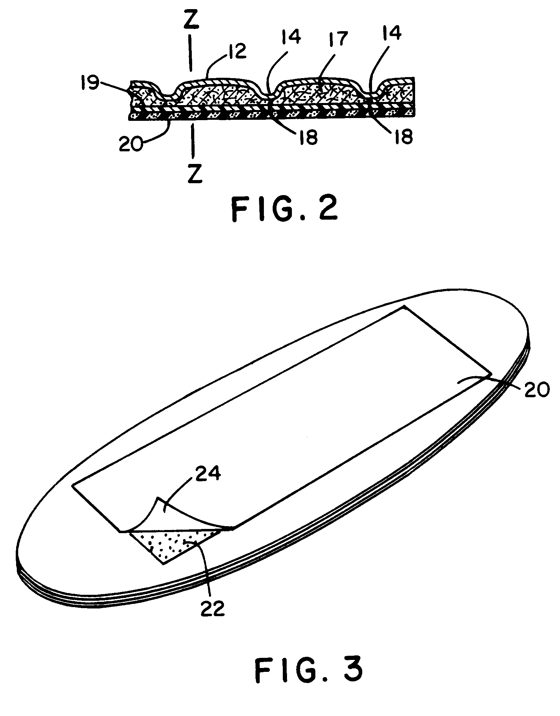

Referring to FIGS. 1 and 2, an example for an absorbent article 10 is shown which is in the form of a panty liner is shown. Although not depicted, other absorbent articles contemplated to be within the scope of the invention include diapers, adult incontinent garments, training pants, sanitary napkins, and the like. The panty liner 10 has a top cover 12 of a nonwoven material with a pattern of spaced longitudinally extending line embossments 14 impressed therein. The line embossments 14 extend the full length of the panty liner 10 and intersect the end and edges of the panty liner 10 at intervals indicated at 15, leaving major portions 16 of the periphery unembossed and therefore unbonded in the z-direction (see FIG. 2). An absorbent 17 consisting of absorbent fibers is positioned below the cover 12 and is compressed along the line embossments 14 which provide hydrogen bonding of the fibers in areas 18. It is this hydrogen bonding which provides the absorbent 17 and the cover 12 with sufficient integrity to prevent delamination in the z-direction. The cover 12 is puffed up and has rounded cushion-like contours between the line embossments 14 where uncompressed and unbonded portions of the absorbent 17 press upwardly against the cover 12. An adhesive, preferably a hot melt adhesive 19 is sprayed onto the absorbent 17 and a foam baffle 20 is adhesively attached thereto.

The nonwoven cover 12 may be any of the many known types of liquid-pervious, nonwoven materials that are liquid-permeable and non-irritating to the skin. For example, the cover 12 may be made from a carded web of polyester, polypropylene, nylon, or other heat bondable fibers. A preferred material is a nonwoven web of stable linked rayon fibers. A more preferred material is a spunbond polypropylene fabric. Spunbonded webs of continuous filaments such as polyethylene, polypropylene, polyester, nylon and the like retain a much drier surface when in use. The most preferred materials are spunbond polypropylene webs having a basis weight from about 10 g/m² to about 40 g/m². A spunbond material containing a whitener such as titanium dioxide (TiO₂) or calcium carbonate (CaCO₃) is desirable because the color exhibits good stain masking properties to hide body fluid that has permeated into the absorbent 17. Spunbond also has sufficient strength so that the cover 12 does not tear or fall apart in use.

The absorbent 17 may be of any desired moisture absorbent material. Typical materials include wood pulp, cotton, rayon, polyesters, coforms, and combinations of these absorbent fibers. A preferred material is devilizated wood fiber fluff as it is low in cost. The fluff may further contain crosslinked highly absorbent polymers ordinarily referred to as superabsorbents either as a separate layer or mixed with the fibers. Preferably, the fibers have a length of from about 0.5 to about 10 millimeters. Many methods for making absorbents are known in the art, so there is no need to describe such method here.

The line embossing 14 binds the short wood pulp fibers in the absorbent 17 to the cover 12 by means of hydrogen bonding and heat. Hydrogen bonding of wood pulp fibers are bonds which develop when the hydroxyl groups of adjacent fibrillae in the cellulosic fibers form hydrogen bonds when the web dries. In this instance, the bonds are formed during the embossing process. Such bonding serves as a means for maintaining the strength and integrity of the absorbent 17 in the z-direction. Sufficient strength and integrity in the x and y directions are provided by adhesively attaching the foam baffle 20 to the absorbent 17. The x, y and z designations are used herein in the customary manner, see FIGS. 1 and 2, i.e. x and y are the longitudinal and traverse directions respectively, while the z-direction is perpendicular thereto. An objective of the line embossing arrangement, in addition to providing the hydrogen bonding which is the primary means for providing z-direction integrity in the panty liner, is to provide the entire surface of the panty liner, including the edges with a soft, cushion-like structure.

Undulating or wavy lines are also preferred for the line embossments 14. When the panty liner is bent into an arcuate shape during use, the wavy or undulating lines permit flexing much more than straight lines. The flexing produces only small scale wrinkles which do not appear to interfere with tactile comfort as large scale wrinkles have been found to do.

The line embossments 14 also direct the migration of the absorbed fluid along the X--X axis. Since the edges of the panty liner are unsealed, it is desirable to keep any of the absorbed liquid away from these edges as long as possible. The longitudinally extending embossments 14 serve this purpose well by acting as conduits which, due to their compressed condition, provide small pores in the absorbent 17 which can enhance capillary suction pressures.

The adhesive 19 can be virtually any adhesive and preferably is any water insoluble or water impermeable hot melt adhesive composition. For convenience, it is generally preferred to employ one of the hot melt adhesives already utilized by the manufacturer in the construction of the particular absorbent article. The hot melt adhesive 19 advantageously has a cold tack to improve its adherence and bonding of the absorbent 17 to the foam baffle 20. The adhesive 19 can be applied by spraying a uniform pattern, atomized with heated air into very small spherical or fiber-like particles, onto the foam baffle 20. Suitable hot melt adhesives are Findley 593-335, an ethylene vinyl acetate blend, and Findley 582-371, as well as various amorphous polypropylenes, all available from Findley Adhesives Inc., located at 11320 Watertown Plank Road, Wauwatosa, Wisconsin. Practical ranges on the amounts of hot melt adhesive to be applied range from about 1 g/m² to about 15 g/m². Preferably, the amount of adhesive ranges from about 5 g/m² to about 12 g/m².

Referring now to FIGS. 2 and 3, the foam baffle 20 may be made from any light weight polyolefin foam material having two or more surfaces capable of being adhesively coated. Such foams may be a closed cell, crosslinked or a non-crosslinked polyolefin foam. Desirably, the foam is a polypropylene or a polyethylene foam, with polyethylene being preferred. Particularly preferred is a non-crosslinked polyethylene foam.

The foam must be flexible and capable of being formed into sheets. The foam should have a thickness, as measured by ASTM D3575, ranging from about 0.51 millimeters to about 6.35 millimeters. Preferably, a thickness of from about 0.51 millimeters to about 1.54 millimeters, more preferably from about 0.76 millimeters to about 1.27 millimeters, and most preferably from about 0.76 millimeters to about 1.02 millimeters.

The foam should have a density ranging from about 0.0225 g/cm³ to about 0.0962 g/cm³. Preferably, the density ranges from about 0.0322 g/cm³ to about 0.0642 g/cm³, and most preferably, from about 0.0354 g/cm³ to about 0.0482 g/cm³. The foam should have the thickness and density measured just after extrusion, prior to any compaction of the foam's cells that may occur through subsequent handling, packaging, or other methods or processes that may alter the foam's characteristics. Examples of commercially available foams contemplated for use in this invention include the trade name CA-30 foam manufactured by Sealed Air Corp. located at 19-01 State Highway 208, Fair Lawn, New Jersey and trade name AF-O30 manufactured by Astro-Valcour, Inc. located at 18 Peck Ave., Glens Falls, New York.

Referring to FIG. 3, disposed on the foam baffle's 20 garment facing surface is a longitudinally extending pressure sensitive adhesive means 22, provided for attaching the panty liner 10 to a crotch portion of an undergarment. While such adhesive means are illustrated in the preferred form of a longitudinal strip, it is understood that various patterns such as spots, or transverse lines will be suitable.

The adhesive employed may be any of the large number of pressure-sensitive adhesives. Non-limiting examples of such adhesives are those hot melt adhesive compositions based on ethylene/vinyl acetate copolymers, isotactic or atactic polypropylene, styrene-butadiene, styrene-isoprene, or styrene-ethylene-butylene A-B-A or A-B-A-B block copolymers. Such adhesives are sold under the Kraton®, Solprene® and Stereon® trade names. In addition to a base polymer, the hot melt adhesive is composed of tackifiers, oils and/or waxes as well as conventional additives including stabilizers, antioxidants, pigments and the like. A preferred adhesive is 34-2823 from National Starch and Chemical Co. located at Finderne Ave., Bridgewater, New Jersey.

Generally, the garment attachment adhesive 22 is applied to the peel strip 24 by suitable methods known in the art. For example, slot coating or spraying. Desirably, the amount of adhesive 22 ranges from about 10 g/m² to about 40 g/m², preferably from about 18 g/m² to about 30 g/m² and most preferably from about 18 g/m² to 25 g/m². It should be recognized that even though this amount of adhesive 22 is adequate to anchor a panty liner, greater amounts of adhesive may be required for anchoring particular structures.

A preferred method of bonding the garment adhesive 22 to the foam baffle 20 is disclosed in the commonly assigned, copending, patent application U.S. Ser. No. 055,052 filed on even date herewith. This disclosure is incorporated by reference and made a part hereof, and a copy of which is enclosed.

Overlying the adhesive 22 is the protective peel strip 24 which is provided to protect the adhesive 22 from contamination and unintended adhesion prior to use. A particularly useful material is a semi-bleached Kraft paper, the adhesive contacting side of which has been silicone treated to provide easy release from the adhesive elements.

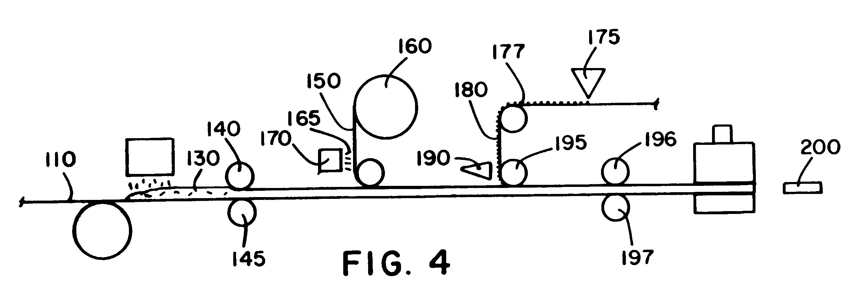

Referring to FIG. 4, a suitable method of fabricating the panty liner 10 will now be described. A liquid-pervious cover 110 has a thin, uniform layer of absorbent 130 air-laid onto one surface thereof. The cover 110 and the layer of absorbent 130 are pressed between two rollers 140 and 145 to densify the absorbent. Optionally, the rollers 140 and 145 may supply sufficient pressure, and heat if required, to hydrogen bond the cover 110 to the absorbent 130 as well as to emboss a pattern thereon. A foam baffle 150 is unwound from a supply roll 160 and sprayed with a hot melt construction adhesive 165 from a sprayer 170. Although not shown, alternatively, the hot melt adhesive 165 may be sprayed onto the absorbent 130 prior to contacting it with the foam baffle 150. The foam baffle 150 is applied to the absorbent 130. An applicator 175 applies a garment attachment adhesive 177 onto one surface of peel strip 180. The adhesive 177 is heated by a heater 190 so that when the adhesive 177 contacts the foam baffle 150 the adhesive 177 will be properly melted to permit bonding. Although not necessary, bonding of the garment attachment adhesive 177 may be enhanced by one or more compression rollers 195, 196 and 197. After bonding, the laminate (consisting of the cover 110, the absorbent 130, the adhesive 165, the foam baffle 150, the garment attachment adhesive 177 and the peel strip 180) is die cut into a desired shape 200 and packaged for shipment.

In using the panty liner of this invention it is preferred that the peel strip 24 be removed from the foam baffle 20 exposing the garment attachment adhesive 22. The panty liner 10 is then centered and mounted in the crotch portion of an undergarment in the usual way.

Panty liners constructed in accordance with the present invention have been found to possess a high liquid absorption capacity. In addition, the panty liner is thin, soft and flexible so it is comfortable to the wearer.

Another advantage is that the panty liner is resilient and conforms well to the surface of the body to which it is applied to achieve good gasketing effects.

While the invention has been described in conjunction a specific embodiment, it is to be understood that many alternatives, modifications and variations will be apparent to those skilled in the art in light of the foregoing description. For example, a wide variety of absorbent products including baby diapers, adult incontinent garments, training pants, wound dressing, sanitary napkins, and the like can incorporate this invention.

## Claims

1. An absorbent article (10) comprising:
(a) a liquid-permeable cover (12;110);
(b) an absorbent (17;130);
(c) a liquid-impermeable foam baffle (20;150) having a thickness ranging from 0.51 millimeters to 1.54 millimeters, and having a density of from 0.0225 g/cm³ to 0.0962 g/cm³, said foam having an absorbent facing surface and a garment facing surface; and
(d) a layer of adhesive (19;165) applied to said absorbent facing surface to bond said absorbent (17;130) to said foam (20;150).

2. The article of claim 1 wherein said foam (20;150) is a polyolefin foam.

3. An absorbent article (10) comprising:
(a) a liquid-permeable cover (12;110);
(b) an absorbent (17;130);
(c) a polyolefin foam baffle (20;150) having a thickness ranging from 0.51 millimeters to 1.54 millimeters, and having a density of from 0.0225 g/cm³ to 0.0962 g/cm³, said foam having an absorbent facing surface and a garment facing surface; and
(d) a layer of adhesive (19;165) applied to said absorbent facing surface to bond said absorbent (17;130) to said foam (20;150).

4. The absorbent article of claim 3 wherein said foam baffle (20;150) is liquid impermeable.

5. The absorbent article of any one of the preceding claims wherein the layer of adhesive (19;165) applied to said absorbent facing surface is a hot melt adhesive.

6. The article of any of claims 2 to 5 wherein said polyolefin foam is polypropylene or polyethylene.

7. The article of any one of the preceding claims wherein said foam (20;150) has a thickness ranging from 0.76 millimeters to 1.27 millimeters and/or has a density of from 0.0322 g/cm³ to 0.0642 g/cm³.

8. The article of claim 7 wherein said foam has a thickness ranging from 0.76 millimeters to 1.02 millimeters and/or has a density of from 0.0354 g/cm³ to 0.0482 g/cm³.

9. The article of any one of the preceding claims wherein the amount of adhesive (19;165) applied to the foam (20;150) ranges from 1 g/m² to 15 g/m².

10. The article of claim 9 wherein the amount of adhesive (19;165) applied to the foam (20;150) ranges from 5 g/m² to 12 g/m².

11. The article of any one of the preceding claims further including:
an amount of pressure-sensitive, garment adhesive (22;177) ranging from 10 g/m² to 40 g/m² applied to said garment facing surface; and
a peel strip (24;180).

12. The article of any one of the preceding claims wherein said article is a panty liner.

13. The article of any one of the preceding claims wherein said cover (12;110) and said absorbent (17;130) are bonded via embossments (14).

14. The article of claim 12 wherein said embossments are line embossments (14) extending preferably in the length direction of the article.

15. An absorbent article (10) comprising:
(a) a liquid-permeable cover (12);
(b) an absorbent (17);
(c) a liquid-impermeable, polyethylene foam baffle (20) having a thickness ranging from 0.76 millimeters to 1.02 millimeters and having a density of from 0.0354 g/cm³ to 0.0482 g/cm³, said foam having an absorbent facing surface and a garment facing surface;
(d) a layer (19) of hot melt adhesive applied to said absorbent facing surface to bond said absorbent to said foam; and
(e) the absorbent article being a panty liner.

16. The article of claim 14 further including:
(f) an amount of pressure-sensitive, garment adhesive (22) ranging from 10 g/m² to 40 g/m² applied to said garment facing surface; and
(g) a peel strip (24).
